# EUROPEAN PATENT SPECIFICATION

(11) **EP 2 139 558 B1**
(45) Date of publication and mention of the grant of the patent: **05.09.2012**
(21) Application number: 07724332.7
(22) Date of filing: 18.04.2007
(51) Int. Cl.: A61N 5/10

(54) **RADIOTHERAPEUTIC APPARATUS**
RADIOTHERAPEUTISCHES GERÄT
APPAREIL RADIOTHERAPEUTIQUE

(43) Date of publication of application: 06.01.2010
(73) Proprietor: Elekta AB (PUBL), 103 93 Stockholm (SE)
(72) Inventor: BROWN, Kevin, John, Horsham West Sussex RH13 5EJ (GB)
(74) Representative: Downing, Michael Philip
(86) International application number: PCT/EP2007/003395
(87) International publication number: WO 2008/128551

(56) References cited:
- WO-A-98/02091
- WO-A-03/076003
- WO-A-2004/004829
- WO-A-2005/024721
- WO-A-2007/016022

## Description

### FIELD OF THE INVENTION

The present invention relates to apparatus and methods for radiation treatment.

### BACKGROUND ART

The Leksel^{™} Gamma Knife^{™} (LGK), and the more recently released Perfexion^{™} system, are radiation treatment systems that employ a large number of individual radiation sources that are arrayed in a hemisphere, and are all collimated to direct a beam of radiation at a single location. As a result, they create a high dose of radiation in a substantially spherical volume around that location and only a low background dose around that volume. Shutters for the sources enable a patient to be moved into position and a prescribed dose of radiation to be delivered to a defined volume within the patient. Treatment volumes that are not spherical or that are larger than the defined volume are dealt with by repeated doses that build up to the prescribed dose.

Until recently, the LGK has been used mainly to treat the head area of a patient, due to volume limitations that flow from the hemispherical structure that carries the sources. More recent versions of the LGK however (in particular the Perfexion^{™} system) are designed for a larger treatable volume and are able to treat regions such as the neck and upper spine.

Existing systems have allowed for the distribution of dose locations to be corrected by way of what is known as a 'rigid body' transformation in which all the dose locations are translated by the same amount to take account of movement of the skull between an Initial imaging step and the treatment.

WO 03/076003 discloses a method in which contoured-anatomy dose repositioning issued to optimize the rigid-body repositioning of the patient so that the daily dose distribution closely matches the planned dose distribution.

WO 2007/016022 and WO 2004/004829 disclose similar methods utilizing rigid-body transformations.

### SUMMARY OF THE INVENTION

The extension of the potential treatment volume to include the head and neck introduces a potential problem, in that these areas are apt to display a greater degree of variance with time as a result of movement by the patient. This leads to deformation of the treatment volume as well as translation. This is not a problem for regions inside the skull as no deformations take place.

Previous techniques based on 'rigid body' transformations are not useful since translating all the dose locations by the same amount is not generally appropriate for the head and neck regions as a result of the potential for deformation as well as translation, a so called 'non-rigid body' transformation. A generic non-rigid body transformation is however too complex to perform swiftly enough for radiation treatment applications.
The present invention therefore provides a treatment planning apparatus for radiation treatment, adapted to accept a treatment plan comprising (I) a prior image of a region to be treated and a plurality of dose locations within the prior image, and (ii) a current image of the region to be treated, the apparatus comprising an associating means arranged, for each dose location, to locate a respective anatomical structure in the prior image proximate that dose location; a comparator for comparing the prior image and the current image, locating In the current image at least those anatomical structures that are associated with a dose location, and determining a transformation between the prior image and the current image for each anatomical structure; and a processing means for determining a current dose location by applying to each dose location the transformation determined in respect of the associated anatomical structure.

This is simply not possible in relation to other types of radiation treatment in which a shaped beam is directed at an isocentre within the patient from a range of different directions as its source rotates around the isocentre. The radiation from such a delivery method does not arrive as a plurality of dose locations; it arrives as a plurality of beam paths that pass through the patient.

The prior image and the current image are ideally three-dimensional, such as from cone beam computed tomography scanning.

The anatomical structures in the prior image can be located through manual identification by a user, or they can be identified using an automated image analysis tool. An example of such a tool is one which allows selection of a number of predefined volumes of the prior image, including and surrounding each dose location.

The associating means and the comparator can be in the form of programs executed by the same processing means. This allows the entire process to be integrated into a single computing device. Alternatively, the tasks can be spread over a number of such computing devices in order to speed processing.

The invention further provides a treatment planning apparatus as defined above associated with a radiotherapeutic apparatus comprising a plurality of individual radiation sources arrayed in a hemisphere, each source being collimated to direct a beam of radiation at a single location within the hemisphere. The radiation sources are preferably decaying isotopes. The Leksell Gamma Knife is an example of such an apparatus.

This invention is particularly useful for the neck region of a patient, where the likelihood and magnitude of non-rigid movements are both high, but there are plenty of distinguishable anatomical features in the form of the vertebrae and air/tissue interfaces.

### BRIEF DESCRIPTION OF THE DRAWINGS

An embodiment of the present invention will now be described by way of example, with reference to the accompanying figures in which;
Figure 1 shows a schematic illustration of a neck region with a planned treatment; and
Figure 2 shows the same neck region after movement.

### DETAILED DESCRIPTION OF THE EMBODIMENTS

Developments in relation to imaging during the time of treatment, generally known as Image Guided RadioTherapy (IGRT), have made available information not only about the displacement of the patient but also the local deformation of non rigid structures. Such deformations can be significant, such as in the neck region. When a radiation dose is delivered using a shaped field (such as a linear accelerator fitted with a Multi-Leaf Collimator), the necessary modification of the treatment to accommodate these deformations is quite complex. However, when the dose is delivered using a sequence of near spherical 'shots' (such as in the LGK) then the required modifications to the dose distribution can be more straightforward.

With the LGK or Perfexion, the dose is delivered by a sequence of shots. Each shot has a predefined position in the treatment planning image. According to the present invention, we displace each individual shot by the local displacement of the anatomy at that position in the image, rather than displace the entire plan or set of shots by the same amount.

This is particularly applicable in the neck, which is non rigid and can be subject to significant deformations between the planning image and the time of treatment. Fortunately, the neck has lots of visible anatomy such as the neck vertebrae, which makes it possible to determine the local displacements. This could be done performing a rigid body registration using the anatomy local to the planned position for the shot, or by performing one deformable registration of the whole image set and then determining the displacements from the result.

This is enabled by the use of 3D imaging e.g. cone beam computed tomography (CBCT) at the time of treatment. It is a simple way of avoiding a complete re-plan of the entire treatment, yet will keep each shot delivered to the right place in the anatomy.

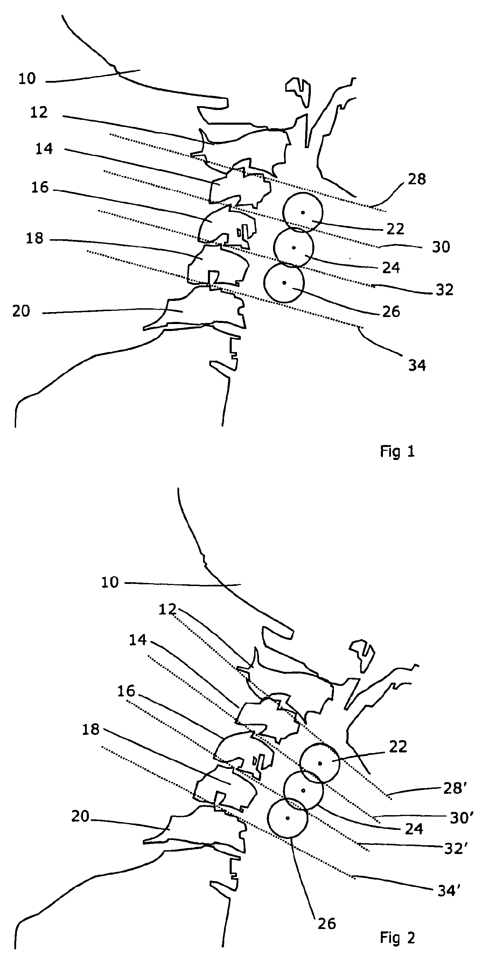

Figures 1 and 2 show the process. Figure 1 is the prior image, projected as a two-dimensional slice for reasons of clarity. A section of skull 10 is clearly visible, together with successive vertebrae 12, 14, 16, 18 and 20. A series of three doses 22, 24 and 26 have been prescribed and these are shown in front of the line of vertebrae. The first step is to locate a visible anatomical feature near to each of the dose locations; in the simple example shown in figure 1 this is clearly the vertebrae nearest each dose location and the associations can be made as follows:

| Dose location | Feature |
|---|---|
| 22 | 14 |
| 24 | 16 |
| 26 | 18 |

An automated image analysis algorithm will however choose image features from the specific image to hand. This could be supplemented or replaced by a user-operated manual association function, allowing each dose location to be selected together with an image feature to associate therewith.

Figure 2 shows the current image of the same patient. Between the two images, the patient has moved his neck, by tilting his head forwards. This results in each vertebrae being rotated by a few degrees and moved slightly.

Guidelines 28, 30, 32 and 34 are positioned in figure 1 relative to the vertebrae 12, 14, 16 and 18, to indicate the position and orientation of the vertebra concerned. It can be seen that the corresponding guidelines 28', 30', 32' and 34' in figure 2 are angled relative to each other, showing the movement of the vertebrae. This movement can be detected by image analysis algorithms that compare the prior and the current image to yield a displacement vector and a rotation representing the movement of an image feature as between the two images.

Accordingly, the apparatus of the invention can then apply the same movement to each dose location as is applicable to the associated image feature. A change that is non-uniform over the image volume is therefore accommodated automatically and without the need for complex processing.

There will be a second order effect in that the applied displacements will change the local density of shots which will change the delivered dose. For example, comparing figures 1 and 2 shows that the overlap between adjacent doses is greater in figure 2 than in figure 1. The magnitude of this effect will depend on the magnitude of the displacements, and may be below a threshold of significance. If not, it can be corrected by adjusting the applied dose at each shot by the change in shot density or a simple function thereof.

This technique is ideally suited to LGK type delivery, where each shot delivers its dose primarily to a single anatomical region, but might also apply to other radiation treatment apparatus that deliver the overall dose as a series of discrete localised sub-doses.

It will of course be understood that many variations may be made to the above-described embodiment without departing from the scope of the present invention.

## Claims

1. A treatment planning apparatus for radiation treatment, adapted to accept a treatment plan comprising (i) a prior image of a region to be treated and a plurality of dose locations within the prior image, and (II) a current image of the region to be treated, the apparatus comprising:
an associating means arranged to locate, for each of the plurality of dose locations, an anatomical structure in the prior image proximate that dose location and to associate said anatomical structure with that dose location;
a comparator adapted to compare the prior image and the current image, to locate in the current image a plurality of anatomical structures associated with the plurality of dose locations, and to determine a transformation between the prior image and the current image for each of the plurality of anatomical structures;
a processing means adapted to determine a current dose location set by:
for each dose location, invoking the associating means, specifying that dose location, and
applying to each dose location the transformation determined in respect of the associated anatomical structure.

2. A treatment planning apparatus according to claim 1 in which the prior image and the current image are three-dimensional.

3. A treatment planning apparatus according to claim 2 in which the prior image and the current image are the result of cone beam computed tomography scanning.

4. A treatment planning apparatus according to any one of the preceding claims in which the anatomical structures in the prior image are located through manual identification by a user.

5. A treatment planning apparatus according to any one of claims 1 to 3 in which the anatomical structures in the prior image are located through automated image analysis.

6. A treatment planning apparatus according to any one of the preceding claims in which the associating means and the comparator are programs executed by the processing means.

7. A treatment planning apparatus according to any one of the preceding claims associated with a radiotherapeutic apparatus comprising a plurality of Individual radiation sources arrayed in a hemisphere, each source being collimated to direct a beam of radiation at a single location within the hemisphere.

8. A treatment planning apparatus according to claim 7 in which the radiation sources are decaying isotopes.

9. A treatment planning apparatus according to any one of the preceding claims in which the prior image and the current image are images of the neck region of a patient.

10. A treatment planning apparatus according to claim 9 in which the anatomical structures include neck vertebrae.

## Patentansprüche

1. Behandlungsplanungsgerät zur Bestrahlungstherapie, das dazu ist, einen Behandlungspfan abzunehmen, aufweisend (1) ein bekanntes Bild eines zu behandelnden Bereichs und eine Vielzahl von Dosispunkten innerhalb des bekannten Bildes, und (ii) ein aktuelles Bild des zu Bereiches, wobei das Gerät aufweist:
eine Zuordnungseinrichtung, die so angeordnet ist, dass sie für jeden der Vielzahl von Dosispunkten in dem bekannten Bild eine anatomische Struktur nahe dieses Dosispunktes ortet und die anatomische Struktur diesem Dosispunkt zuordnet;
einen Komparator, der dazu geeignet ist, das bekannte Bild und das aktuelle Bild zu vergleichen, in dem aktuellen Bild eine Vielzahl von der Vielzahl von Dosispunkten zugeordneten anatomischen Strukturen zu orten, und für jede der Vielzahl von anatomischen Strukturen eine Transformation zwischen dem bekannten Bild und dem aktuellen Bild zu bestimmen;
eine Verarbeitungseinrichtung, die dazu geeignet ist, einen aktuellen Dosispunkt zu bestimmen, eingestellt durch:
das Spezifizieren des Dosispunktes für jeden Dosispunkt unter Aktivierung der Zuordnungseinrichtung, und
Anwenden der bezüglich der zugeordneten anatomischen Struktur Transformation auf jeden Dosispunkt.

2. Behandlungsplanungsgerät nach Anspruch 1, bei dem das bekannte Bild und das aktuelle Bild dreidimensional sind.

3. Behandlungsplanungsgerät nach Anspruch 2, bei dem das bekannte Bild und das aktuelle Bild das Ergebnis eines Scanning mit Kegelstrahl-Computertomographie ist.

4. Behandlungsplanungsgerät nach einem der vorhergehenden Ansprüche, bei dem die anatomischen Strukturen in dem bekannten Bild durch manuelle Erkennung durch einen Benutzer geortet werden.

5. Behandlungsplanungsgerät nach einem der Ansprüche 1 bis 3, bei dem die anatomischen Strukturen in dem bekannten Bild durch automatisierte Bildanalyse geortet werden.

6. Behandlungsplanungsgerät nach einem der vorhergehenden Ansprüche, bei dem die Zuordnungsmittel und der Komparator Programme sind, die von der Verarbeitungseinrichtung ausgeführt werden.

7. Behandlungsplanungsgerät nach einem der vorhergehenden Ansprüche, das einem radiotherapeutischen Gerät zugeordnet ist, das eine Vielzahl von individuellen Strahlungsquellen, die in einer Hemisphäre angeordnet sind, aufweist, wobei jede Quelle parallel gerichtet wird, um einen Bestrahlungsstrahl auf einen einzelnen Punkt innerhalb der Hemisphäre zu richten.

8. Behandlungsplanungsgerät nach Anspruch 7, bei dem die Strahlungsquellen zerfallende Isotope sind.

9. Behandlungsplanungsgerät nach einem der vorhergehenden Ansprüche, bei das bekannte Bild und das aktuelle Bild Bilder des Hals- bzw. Nackenbereichs eines Patienten sind.

10. Behandlungsplanungsgerät nach Anspruch 9, bei dem die anatomischen Strukturen Halswirbelknochen umfassen.

## Revendications

1. Appareil de planification de traitement pour un traitement par radiothérapie, adapté à accepter un plan de traitement comprenant (i) une image précédente d'une région à traiter et une pluralité d'emplacements de dose au sein de l'image précédente, et (ii) une image courante de la région à traiter, l'appareil comprenant :
un moyen d'association agencé pour localiser, pour chacun de la pluralité d'emplacements de dose, une structure anatomique dans l'image précédente à proximité de cet emplacement de dose et pour associer ladite structure anatomique avec cet emplacement de dose ;
un comparateur adapté à comparer l'image précédente et l'image courante, à localiser dans l'image courante une pluralité de structures anatomiques associées avec la pluralité d'emplacements de dose, et à déterminer une transformation entre l'image précédente et l'image courante pour chacune de la pluralité de structures anatomiques ;
un moyen de traitement adapté à déterminer un emplacement de dose courant défini en :
pour chaque emplacement de dose, en invoquant le moyen d'association, spécifiant cet emplacement de dose, et
appliquant à chaque emplacement de dose la transformation déterminée pour ce qui concerne la structure anatomique associée.

2. Appareil de planification de traitement selon la revendication 1 dans lequel l'image précédente et l'image courante sont tridimensionnelles.

3. Appareil de planification de traitement selon la revendication 2 dans lequel l'image précédente et l'image courante sont le résultat d'une topographie à faisceau conique assistée par ordinateur.

4. Appareil de planification de traitement selon l'une quelconque des revendications précédentes dans lequel les structures anatomiques dans l'image précédente sont localisées par une identification manuelle par un utilisateur.

5. Appareil de planification de traitement selon l'une quelconque des revendications 1 à 3 dans lequel les structures anatomiques dans l'image précédente sont localisées par une analyse automatisée d'images.

6. Appareil de planification de traitement selon l'une quelconque des revendications précédentes dans lequel le moyen d'association et le comparateur sont des programmes exécutés par le moyen de traitement.

7. Appareil de planification de traitement selon l'une quelconque des revendications précédentes associé avec un appareil de radiothérapie comprenant une pluralité de sources individuelles de rayonnement rassemblées en un hémisphère, chaque source étant collimatée pour diriger un faisceau de rayons sur un emplacement unique au sein de l'hémisphère.

8. Appareil de planification de traitement selon la revendication 7 dans lequel les sources de rayonnement sont des isotopes en voie de désintégration.

9. Appareil de planification de traitement selon l'une quelconque des revendications précédentes dans lequel l'image précédente et l'image courante sont des images de la région de cou d'un patient.

10. Appareil de planification de traitement selon la revendication 9 dans lequel les structures anatomiques incluent vertèbres cervicales.
